# EUROPEAN PATENT APPLICATION

(11) **EP 4 434 551 A1**
(43) Date of publication of application: **25.09.2024**
(21) Application number: 23163555.8
(22) Date of filing: 22.03.2023
(51) Int. Cl.: A61K 51/00, A61P 35/00, A61P 35/04, G01N 33/574, A61K 101/02

(54) **METHOD FOR DIAGNOSING AND TREATING NIS-EXPRESSING CARCINOMAS AND METASTASES**

(71) Applicant: Nuske, Andreas, 78166 Donaueschingen (DE)
(72) Inventor: The designation of the inventor has not yet been filed
(74) Representative: dompatent von Kreisler Selting Werner - Partnerschaft von Patent- und Rechtsanwälten mbB

(57) **Abstract**

The present invention relates to a method for diagnosing and/or treating sodium-iodide symporter (NIS)-expressing primary carcinomas and metastases, preferably glandular carcinomas, in particular carcinomas of the thyroid, of the salivary gland, of the uterus and carcinomas of the breast, and to a pharmacological composition comprising substances which induce and/or increase the expression or function of the NIS symporter and, as a consequence, increase iodide uptake into the cells, and to corresponding uses. This can be used for an efficient tumor-specific radioiodide uptake in diagnosis and therapy of said carcinomas and metastases.

## Description

The present invention relates to a method for diagnosing and/or treating sodium-iodide symporter (NIS)-expressing primary carcinomas and metastases, preferably glandular carcinomas, in particular carcinomas of the thyroid, of the salivary gland, of the uterus and carcinomas of the breast, and to a pharmacological composition comprising substances which induce and/or increase the expression or function of NIS and, as a consequence, increase iodide uptake into the cells, and to corresponding uses. This is used for an efficient organ-specific radioiodide uptake in diagnosis and therapy of said carcinomas and metastases.

### Background of the Invention

Some methods for diagnosing and treating primary glandular carcinomas and metastases of glandular carcinomas such as of the thyroid, of the salivary gland, of the uterus and carcinomas of the breast are known in the art. Known diagnostic methods include sonography, mammography and magnetic resonance imaging. The disadvantages of these methods are in particular that they must be employed in combination in order to increase the specificity of the diagnosis. Ordinarily, a core needle biopsy follows and may lead to dissemination of cancer cells and thus to iatrogenic metastasis. The definitive diagnosis must be made by surgical resection or core needle biopsy and histological examination of the nodule, and it often emerges that the nodule is benign. The elaborate diagnostic procedure frequently results in loss of time and unnecessary surgical procedures.

Primary therapy of said tumors usually consists of surgical resection of the primary carcinoma. Depending on the size of the tumor (e.g., larger than 2 cm in diameter) or evidence of lymph node involvement, especially in carcinoma of the breast, additionally the ipsilateral axillary lymph nodes are removed, with, where appropriate, irradiation of the supraregional area. Investigations are performed postoperatively to detect or exclude remote metastases, i.e., for example an upper abdominal sonography, a chest radiograph and a skeletal scintigram. Only metastases above a certain size are identified in these cases. Smaller metastases, i.e. for example smaller than 0.5 cm in diameter, so-called micrometastases, are often not identified. In the bone scintigram, degenerative or inflammatory changes may lead to false-positive findings. In the event of metastases, chemotherapy is carried out, but may have considerable side effects. This treatment is only palliative and leads merely to a small increase in lifespan.

According to statistics published in 2020 by the Center for Cancer Registry Data of the Robert Koch Institute, breast cancer is by far the most common carcinoma entity and the most common cause of cancer death among women in Germany, with approximately 70,000 new cases annually. Every eighth to tenth woman suffers in the course of her life from breast cancer, half of the women affected before the age of 65. Every tenth even before the age of 45, the prognosis for a mamacarcinoma diagnosis remains relatively improvable despite multiple treatment options, such as mastectomy, lumpectomy, radiation, hormonal therapy and chemotherapy. In 2010, the five-year survival rate was 79.6%; in 2019, it was 84%. Although the survival rate is increasing, the immense number of new disease as well as the continued poor prognosis in metastatic disease (life expectancy max. about 48 months), which makes the need to develop new therapeutics or therapeutic approaches a public health priority more than clear. Based on gene expression of standard molecular markers such as estrogen receptor α (ERα), progesterone receptor (PR) and human epidermal growth factor receptor 2 (HER2), breast cancer is classified into four main subtypes: Luminal A (ERα and/or PR-positive and HER2-negative = *Cell MCF-7* ); Luminal B (Erα and/or PR positive and HER2 positive or negative = *BT474*); HER2-positive (Erα/PR-negative and HER2-positive = *Cell SKBR3*); Triple-negative, TNBC (ERα/PR/HER2-negative = Cell MDA-MB-231), each characterized by different prognosis and response to drug treatment.

Currently, locoregionary (surgery and radiation) and systemic (chemotherapy, endocrine and biological therapies) approaches represent the main therapeutic options for the treatment of breast cancer. Despite significant progress in systemic therapy, particularly with the sometimes quite promising class of monoclonal antibodies, approximately 30% of patients relapse and develop metastatic disease, ultimately leading to death from breast cancer after approximately 48 months from diagnosis. In recent years, also Peroxisom-Proliferator-activated receptor γ (PPAR), a ligand-activated transcription factor mainly characterized as a central regulator of adipocyte differentiation, has received considerable attention because of its role in mammary carcinoma tumorigenesis. Clinical studies have shown that PPAR expression is a positive prognostic factor in patients with luminal and ductal breast cancer, as higher PPAR levels correlate reciprocally with tumor size grade and TNM staging system of the malignant tumor.

For providing a rapid, specific and reliable diagnostic method and an efficient treatment of carcinomas and metastases which express a sodium-iodide symporter (NIS) gene, such as in primary glandular carcinomas and metastases of glandular carcinomas, especially of carcinomas of the thyroid, of the salivary gland, of the uterus, and of carcinomas of the breast, WO 2003/107001 suggest the co-administration of a PPAR-y ligand and at least an RAR ligand and/or an RXR ligand for inducing cell-specific stimulation of enhanced iodide uptake through inducing or increasing NIS gene expression and function in said types of tumors, which uptake can be utilized for specific diagnosis and/or targeted therapy.

The sodium-iodide symporter (NIS) is responsible for active iodide uptake in the thyroid. The iodine is used in the follicle cells of the thyroid for the purpose of biosynthesizing thyroid hormones (N. Carrasco, Biochim Biophys Acta. 1993 Jun. 8; 1154 (1): 65-82). NIS expression has been utilized for decades for diagnosing and treating differentiated thyroid carcinomas based on the administration of radioiodide (E. L. Mazzaferri, 1996 in The Thyroid, eds. L. E. Braverman and R. D. Utiger (Lippincott-Raven, Philadelphia), pp. 942-945). NIS is also expressed, in some cases functionally, in many extrathyroid tissues such as kidneys, placenta, salivary glands, gastric mucosa and lactating mammary gland, as has been demonstrated at the mRNA and protein levels (A. De La Vieja et al., Physiol Rev. 2000 July. 80 (3): 1083-105).

NIS has been detected in 80% of human mammary gland cancers by means of immunohistochemistry (U. H. Tazebay et al., Nat Med. 2000 Aug. 6 (8): 871-8), without, however, identifying a biological in vivo function. Breast cancer is the third commonest cancer in the world and the commonest malignant disease of women. The expression of functional NIS may therefore be useful in the radioiodide diagnosis and therapy of responsive tumors of the breast (see reviews by A. De La Vieja et al., Physiol Rev. 2000 July 80 (3): 1083-105; C. Riedel et al., Trends Biochem Sci. 2001 Aug. 26 (8): 490-6; A. E. Heufelder et al., Thyroid. 2001 Sep. 11 (9): 839-47).

Factors which modulate NIS expression and iodide uptake in breast cancer cells have been reported, namely the induction of the NIS gene by trans-retinoic acid (tRA) in the breast cancer cell line MCF-7. It was suggested that the tRA-induced NIS expression is stimulated by two families of nuclear receptors, namely retinoic acid receptors (RARs) and retinoid X receptors (RXRs) (T. Kogai et al., Proc Natl Acad Sci USA. 2000 July 97 (15): 8519-24). MCF-7 cells express not only functional RAR/RXR but also nuclear PPAR-y (peroxisome proliferator activated receptor-y) (M. W. Titcomb et al. Mol Endocrinol. 1994 Jul. 8 (7): 870-7; M. W. Kilgore et al., Mol Cell Endocrinol. 1997 May 129 (2): 229-35). PPAR-y regulates, in the presence of appropriate ligands, through heterodimerization with RAR/RXR (RXRα, RXRβ or RXRy) the transcription of target genes (M. Sato et al. Biochem Biophys Res Commun. 2001 January. 280 (3): 646-51). PPAR-y and RAR/RXR are co-expressed in various malignant epithelial tumors such as carcinomas of the brain, breast, prostate and lung (J. O. Nwankwo and M. E. Robbins, Prostaglandins Leukot Essent Fatty Acids. 2001 April-May 64 (4-5): 241-5; K. Inoue et al., Anticancer Res. 2001 July-August 21 (4A): 2471-6).

In WO 2003/107001 it is shown that iodide uptake in cells of certain carcinomas can be increased by a simultaneous or sequential action of PPAR-y ligands and RAR/RXR ligands. In particular, WO 2003/107001 utilizes a combination of ciglitazone, a synthetic PPAR-y ligand, with retinoic acid as RAR/RXR ligand. However, the increase of the NIS expression is still not sufficient to enable a meaningful treatment or diagnosis of cancer.

It was now found that by additional administration of a glucocorticoid the uptake of a radioactive entity such as radioactive iodine of the relevant cancer cells is significantly increased. The invention thus provides:
(1) A diagnostic composition, therapeutic composition or medicament for use in the *in vivo* diagnosis or treatment of NIS gene-expressing carcinomas and/or metastases of carcinomas by stimulating or enhancing NIS gene expression in cells of the carcinomas or the metastases thereby increasing NIS-symporter function, said composition comprising the components:
   (a) at least one Peroxisome Proliferator-Activated Receptor-y (PPAR-y) ligand,
   (b) at least one Retinoic Acid Receptor (RAR) and/or Retinoid X Receptor (RXR) ligand,
   (c) at least one glucocorticoid, or a salt and/or ester thereof, and
   (d) a radioactive entity actively transported by the NIS symporter into cells of the carcinomas or the metastases.
(2) A preferred embodiment of aspect (1) above, which is a diagnostic composition and is used for diagnosis of carcinomas and/or metastases of carcinomas, wherein the diagnosis comprises
   (a) application of the diagnostic composition as defined in aspect (1) above to a patient in need of such diagnosis;
   (b) detecting the accumulation of the radioactive entity (d) in the body region of the patient suspected to contain the carcinomas or metastases by radio-detection;
   (c) evaluating and comparing the radioactive signals with the radioactive signal of a healthy patient or an earlier signal of the same patient; and
   (d) determining the severity or change of carcinomas or presence of metastases in the patient.
(3) A method for *in vivo* and *ex vivo* diagnosis of NIS gene-expressing carcinomas and/or metastases of carcinomas by stimulating or enhancing NIS gene expression in cells of the carcinomas or the metastases thereby increasing NIS-symporter function, said method comprises administering a patient or subjecting a tissue sample in need of such diagnosing a composition comprising the components:
   (a) at least one Peroxisome Proliferator-Activated Receptor-y (PPAR-y) ligand,
   (b) at least one Retinoic Acid Receptor (RAR) and/or Retinoid X Receptor (RXR) ligand,
   (c) at least one glucocorticoid, or a salt and/or ester thereof, and
   (d) a radioactive entity actively transported by the NIS symporter into cells of the carcinomas or the metastases.
(4) A method for treating NIS gene-expressing carcinomas and/or metastases of carcinomas by stimulating or enhancing NIS gene expression in cells of the carcinomas or the metastases thereby increasing NIS-symporter function, comprising: administering to a patient in need thereof a composition comprising the components:
   (a) at least one Peroxisome Proliferator-Activated Receptor-y (PPAR-y) ligand,
   (b) at least one Retinoic Acid Receptor (RAR) and/or Retinoid X Receptor (RXR) ligand,
   (c) at least one glucocorticoid, or a salt and/or ester thereof, and
   (d) a radioactive entity actively transported by the NIS symporter into cells of the carcinomas or the metastases,
   to stimulate or enhance uptake of the radioactive affinity containing substance actively transported by the NIS symporter by induction of NIS gene expression in the cells of the patient.

### Short Description of the Figures

Figure 1 shows the results of the experiments with MCF-7 cells and the following test conditions: • 0,0% untreated MCF-7, control 3 repetitions; and ▪ 0.1% DMSO, control 3 repetitions.
Figure 2 shows the results of the experiments with MCF-7 cells and the following test substance concentrations: (1) 1 µM atRA, control 5 repetitions; (2) 0.1 µM dexamethasone acetate, control 4 repetitions; (3) 10 µM Pioglitazone, control 4 repetitions; (4) 10 µM DHA900EE, control 3 repetitions.

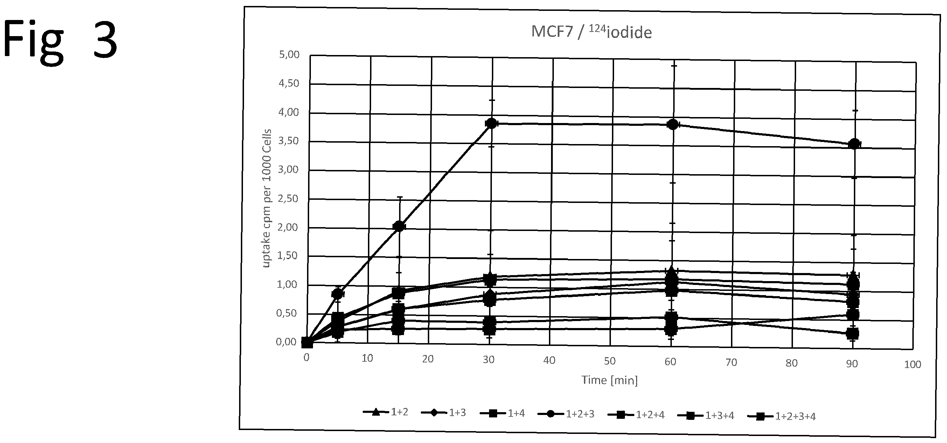

Figure 3: shows the results of the experiments with MCF-7 cells and the following test substance combinations and concentrations: (1+2) 1 µM at RA and 0.1 µM dexamethasone acetate, control 4 repetitions; (1+3) 1 µM atRA and 10 µM Pioglitazone, control 4 repetitions; (1+4) 1 µM atRA and 10 µM DHA900EE, control 4 repetitions; (1+2+3) 1 µM atRA, 0.1 µM dexamethasone acetate and 10 µM Pioglitazone, control 4 repetitions; (1+2+4) 1 µM at RA, 0.1 µM dexamethasone acetate and 10 µM DHA900EE, control 4 repetitions; (1+3+4) 1 µM atRA, 10 µM Pioglitazone, and 10 µM DHA900EE, control 4 repetitions; (1+2+3+4) 1 µM atRA, 0.1 µM dexamethasone acetate, 10 µM Pioglitazone and 10 µM DHA900EE, control 3 repetitions.
Figure 4 shows the results of the comparative experiments with HELA cells and the following test substance combinations and concentrations: (1) 1 µM atRA: (2) 0,1 µM dexamethasone acetate; (3) 10 µM Pio and (4) 10 µM DHA900EE.

### Detailed Description of the Invention

In the studies which were already performed, PPARs were shown to form a subfamily of nuclear hormone receptors (group C of subfamily 1 [ NR1C]) and to represent a ligand-activated transcription factor. Nuclear receptors include receptors for steroid hormones, thyroid hormone, Retinoide, among others. There are several subtypes: PPARα (NR1C1), PPARβ (NR1C2), and PPARy (NR1C3); in the underlying idea, the focus is set on PPAR. Each subtype is encoded by a distinct GEN and is characterized by a highly specific expression pattern. The human PPARy gene (peroxisome proliferator-activated receptor gamma Homo sapiens - Gene - NCBI (nih.gov); SEQ ID NO:1), NR1C3, is located on chromosome 3p25.2 and contains approximately 9 exons that result in the above three differential PPARy transcripts (γ1, y2, and y3) through alternative promoter usage and splicing. PPARγ1 and PPARy3 mRNAs encode the same protein (SEQ ID NO:2), whereas the PPARy2 mRNA protein (SEQ ID NO:3) contains 30 additional amino acids in the N-terminal region. PPARγ1 mRNA has ubiquitous expression, although the highest levels were found in adipose tissue; PPARy2 mRNA is mainly expressed in adipocytes; PPARy3 mRNA was frequently found in macrophages and adipose tissue. However, regardless of PPAR subtype, several cell types express different levels of PPARy.

Interestingly, PPARy is also expressed in various types of tumors, especially glandular tumors, preferably in breast, prostate and thyroid cancer, so it is highly likely that our applied stimulants or agants, preferably the combination of these mono-test substances, can also successfully stimulate NIS expression in glandular tumor cells, thus, with the help of [¹²⁴I]iodide (I-124) and [¹³¹I]iodide (I-131)], in vitro and/or in vivo diagnostics (e.g. scintigraphy) and/or medicinal (a kind of "Trojan Horse") therapy is crowned with success.

However, PPARy was not found only in epithelial cancer cells but also in several components of the tumor microenvironment, such as tumor-associated macrophages (TAMs) and cancer-associated fibroblasts (CAFs), which suggests that PPARy may be a good target for integrative therapies which simultaneously affect tumors and their microenvironment. Like other members of the nuclear receptor family, PPARy activity depends on its intracellular localization. In the absence of specific acting ligands, PPARy is localized in the cytoplasm bound to transcriptional corepressor complexes containing the nuclear receptor corepressor complexes or the Retinoic Acid and Thyroid Hormone silencing mediator, which prevents the activation of PPARy.

The activation of the gene expression requires precisely these PPARy-specific ligands (among others Thiazolidinedione (TZDs)), which interestingly can cooperate with RXR ligands (among others all-trans retinoic acid (atRA)) to modulate the transcription of PPARy target genes and then form a heterodimer with the Retionid-X Receptor (RXR) and thereby influence the transcription rate of numerous genes by binding to the so-called Peroisome proliferator response elements (PPREs) in the promoter region of the target genes. Thus, among others, Insulin sensitization and carcinogenesis are activated or influenced.

In the underlying idea this physiological phenomenon was used and it proved in substantially carried out cell line trials that this archetypically anchored effect is obtained not only by the stimulation of the PPARy's, with the help of which, among other things, specific ligands, the substance class of the synthetic, so-called insulin sensitizer Thiazolidinediones (=TZDs; Pioglitazone was used), but additively by additional stimulation of the Retinoid x-Receptors, with the help of which specific ligands, the substance class of synthetic all-trans-Retinoic Acid (=Retioniden, atRA), is more intensively applied, and surprisingly, the addition of a glucocorticoid (for example dexamethasone or an ester thereof), as the most frequently used glucocorticoid, which actually serves as an agonist of Glucocorticoids-receptor, did not additively increase the additive effect of heterodimers (RXR and PPARy) by stimulating glucocorticoid-receptors, but directly and immediately on the effect of heterodimers and thus on the NIS expression stimulating effect and the uptake of radioiodids significantly increased and thereby brought up a synergistic effect so that the stimulation of the target gene (NIS expression), which then led to the significant induction of specific mRNA (protein synthesis) and the associated expression of the, for the therapeutic benefit guaranteeing, specific sodium-iodide-pump or the endogenous Sodium-Iodine-Symporter (NIS), which is responsible for the uptake of radioiodide after the application of the preferred triple combination of active substances (TZD, atRA), because the majority of breast cancer diseases (70-80%) expresses NIS and because the mammary gland iodide transporter is expressed during lactation and in breast cancer (Tazebay et al., NatMed 6(8): 871-878 (2000)). Whereas the additionally provided cell line trials revealed that untreated MCF-7 cells do not produce NIS and thus correspondingly the uptake of radioiodide has failed in homeopathic level, approximately to the same extent as the use of the applied three computational models "DSMO" mono substance.

Furthermore, the additionally performed cell death experiments, on the one hand with also malignant breast cancer cells achieved only a moderate uptake of radioiodide and on the other hand with HeLa cell line, which is a non-NIS expressing cervical carcinoma cell line, which did not express NIS at all and thus correspondingly achieved only a homeopathic uptake of the radioiodide ¹²⁴I (I-124) used, which served as a negative control. Thus, the Specificity of MCF-7 cell lines using the test compounds described above was impressively demonstrated. Furthermore, to strengthen the Specificity, we still performed blocking attempts (point no. 10) on the MCF-7 - cell line.

The above-mentioned synergism is attributable to a heterodimerization of the PPAR-y and RAR/RXR receptors and an NIS gene expression which is induced or stimulated and/or enhanced thereby. An increase, brought about in this way, of the iodide uptake into the cell is utilized according to the invention for the diagnosis and therapy of NIS-expressing primary carcinomas and metastases, especially primary tumors and/or metastases of glandular carcinomas, especially of carcinomas of the breast, of the salivary gland, of the uterus and of carcinomas of the thyroid, and other glandular carcinomas.

On the basis of the described results, a method for diagnosing and/or treating sodium-iodide symporter (NIS)-expressing carcinomas and/or metastases is proposed according to the invention. In this method there is organ-specific stimulation of an induction or increase in NIS gene expression in said types of tumor and enhanced iodide uptake, which is utilized according to the invention for a specific diagnosis and/or a targeted therapy.

The method of the invention can be employed particularly preferably for the diagnosis and/or treatment of glandular carcinomas, especially carcinomas of the salivary gland, of the thyroid, of the uterus and in particular of breast cancer or carcinomas of the breast, and/or the corresponding metastases.

The induced or increased NIS gene expression is utilized according to the invention for introducing, through the enhanced expression or function of the sodium-iodide symporter, preferably radioactive substances into the tumor cells. The radioactivity can be utilized as a very sensitive indicator in the diagnosis by means of scintigraphic methods and/or as active therapeutic agent in the therapy by means of radiolysis. Radiolysis means destruction of the cells by radioactive radiation. A particular advantage of radiolysis is that, even with only a very small uptake of radioiodide, the radiolytic effect is, owing to the range of the radiation, transferred to neighboring cells as so-called bystander effect.

The substances preferably employed according to the invention are those having a high affinity for the NIS symporter, especially iodine and/or technetium. However, other substances with affinity, especially from the halogen group, can also be used. The use of radioiodide and/or technetium, especially radioactive technetium, after preferably systemic use of the NIS gene-inducing active compounds, i.e. in particular the PPAR-y and RAR/RXR ligands, proves according to the invention to be particularly valuable means for the diagnosis and treatment of the tumors indicated above. It is possible and particularly advantageous to use as radioactive iodine for this purpose ¹²³I, ¹²⁵I and/or ¹³¹I, especially in the form of an alkali metal or alkaline earth metal iodide, for example sodium iodide (NaI). In this connection, ¹²⁵I is particularly advantageously employed for diagnosis. ¹³¹I is particularly advantageously employed for therapy.

The term "iodide uptake" hereinafter also means uptake of other substances which have an affinity for the NIS symporter and can be actively transported by it. Addition of the substance whose transport by the sodium-iodide symporter is to be detected or which is intended to cause radiolysis advantageously takes place after an appropriate time after administration of the active compounds which serve to stimulate and/or enhance NIS gene expression. An appropriate time may in this connection be for example about 1 to 5 days, preferably about 1 to 3 days. About 2 days are particularly preferred.

In a preferred embodiment of the method of the invention, NIS gene expression is induced by means of a treatment with at least one PPAR ligand, especially a PPAR-y ligand, at least one RAR/RXR ligand as active compounds, and at least one glucocorticoid in which case the iodide uptake which is increased by the RAR/RXR ligand and PPAR-y ligand is potentiated in a synergistic manner by the glucocorticoid. It is particularly preferred in this connection for the ligands for the two types of receptors to be administered sequentially, with the at least one RAR/RXR ligand preferably being administered first, the at least one PPAR ligand being administered second, and at least one glucocorticoid being administered after an appropriate time. The intention is that initial induction of NIS gene expression takes place in this time. It is additionally possible through the sequential administration to abolish a blockade of the PPAR-y, which may be brought about by a suppressor, by an RAR/RXR activated with an appropriate ligand, so that the PPAR-y ligand can subsequently have a maximal effect. A suitable pretreatment with an RAR/RXR ligand, in particular with retinoic acid, can advantageously be carried out over a few hours up to some days. 1 to 3 days are particularly preferred, especially 2 days. Such a pretreatment can take place for example through an appropriate infusion and/or through single or multiple oral administration of the appropriate active compound. In addition, initial administration of the at least one PPAR-y ligand, sequential administration of the at least one glucocorticoid and the at least one RAR/RXR ligand may also be preferred. Simultaneous administration of ligands for both types of receptors in combination with glucocorticoid may likewise lead to the desired result and be preferred.

Ligands preferably used for the PPAR-y receptor are active compounds from the class of thiazolidinediones, especially ciglitazone, pioglitazone, rosiglitazone, troglitazone or mixtures thereof, or salts thereof. The ligand preferably employed for the RAR/RXR receptors is retinoic acid (RA) or a pharmacologically/diagnostically acceptable derivative or salt thereof, especially all-trans RA (atRA; Tretionin) and/or 9-cis RA (Alitretinoin) and/or 13-cis RA (Isotretinoin), as well as suitable synthetic ligands of retinoic acid receptor (RAR), or a pharmaceutically/diagnostically acceptable derivative or salt thereof. This derivative may be for example a salt or an ester, in particular an ester with an alkanoic acid, preferably with an alkanoic acid having 1 to 4 C-atoms, or an ester with an inorganic acid such as a phosphoric acid or sulfuric acid. The glucocorticoid is selected from natural glucocorticoids, such as cortison and cortisol; synthetic halogenated glucocorticoids, such as amcinonide, alclometasone beclomethasone, betamethasone, clobetasone, clobetasole, cloprednole, clocortolone, dexamethasone, desoximetasone, diflucortolone, fluprednidene, fluocinolone, fluocortolone, flumethasone, halometasone, halcinonide, mometasone and triamcinolone; and synthetic non-halogenated glucocorticoids, such as budesonide methylprednisolone, prednicarbate, prednisone, prednisolone, prednicarbate, prednisone, prednisolone and rimexolone, preferably the glucocorticoid is dexamethasone, dexamethasone acetate or disodium dexamethasone phosphate.

In a further embodiment of the invention, besides the PPAR-y, RAR/RXR ligands and glucocorticoid described above it is also possible to employ additional substances which have a modulating, in particular activating, effect on the activity of the active compounds. These substances are preferably activators, in particular coactivators, which are able for example to form a complex with the two types of receptors. Substances suitable for this purpose are those able to take part as further receptor in the complex formation and which have an activating effect on NIS gene expression in the sense according to the invention.

It is additionally possible by administering suitable substances to attenuate and/or eliminate the effect of cell-intrinsic substances which act in particular as suppressors to inhibit the activation of NIS gene expression by PPAR ligands and RAR/RXR ligands, so that NIS gene expression can be increased according to the invention. Examples of such suppressors which inhibit activation of NIS gene expression are the liver lipid receptor (LXR) and/or the thyroid hormone receptor (Ide T. et al., Molecular Endocrinology, May 2003; Behr M. and U. Loos, Exp Clin Endocrinol Diabetes 1996 (104) Suppl 4: 111-6). These receptors and/or corresponding agonists, e.g. ligands of these receptors, attenuate or suppress the heterodimerization of PPAR-y and RAR/RXR. Administration of antagonists of these receptors having a suppressant effect eliminates or blocks these suppressors according to the invention, and thus enhances the interaction of PPAR-y and RAR/RXR and therefore increases their effect according to the invention on NIS gene expression and function. Examples of antagonists which can be employed are those ligands of the liver lipid receptor and/or thyroid hormone receptor which block the activity thereof. Examples of such antagonists, especially of thyroid hormone receptor antagonists, are TRIAC and/or TETRAC.

A further possibility is also to employ as activator for example a histone deacetylase inhibitor, in particular trichostatin A and/or a butyrate. It is known of histone deacetylase inhibitors that it increases NIS expression in a moderately differentiated thyroid carcinoma cell line (M. Kitazono et al., J. Clin Endocrinol Metab. 2001 July 86 (7): 3430-5). Alternatively, suppressors which downregulate in particular the expression of the NIS gene can be eliminated or blocked by suitable substances.

Targeted induction of NIS gene expression for the diagnosis and/or therapy of said types of tumors can also be achieved according to the invention with other active compounds. These active compounds may be hormones or other ligands for particular receptors which are expressed on the surface of the cells of the indicated metastases or carcinomas. An example which should be mentioned of such active compounds is prolactin, which is known to be able to modulate iodide uptake in lactating mammary gland cells.

A particularly preferred embodiment of the diagnostic method of the invention includes an in vivo diagnosis. For this purpose, at least one ligand for the PPAR-y receptor, e.g. a thiazolidinedione such as, for example, ciglitazone, at least one ligand for the RAR and/or RXR receptor, e.g. trans-retinoic acid, at least one glucocorticoid, e.g. dexamethasone or dexamethasone acetate is administered to the patient to be investigated. Oral or parenteral administration is preferred. Intravenous administration of the substances is likewise possible. Preferably after an appropriate time during which induction of the NIS gene takes place, a suitable dose of a further substance, in particular technetium and/or radioactive iodine, e.g., ¹³¹iodine, is administered. This time is advantageously about 1 to about 5 days, preferably about 1 to about 3 days. About 2 days are particularly preferred. Preferably after a further appropriate period of time, which is suitable for iodide uptake or, for example, technetium uptake in cells, the uptake of the substance into the cells is analyzed. This takes place for example by a whole-body scintigram or a local scintigram, for example of the breast, to detect primary tumors and/or metastases. Since the iodide uptake after such a treatment is potentiated according to the invention only in NIS-expressing cells, the in vivo diagnostic method of the invention makes specific indication of tumors possible. With this method it is possible to detect specifically even very small tumors or micrometastases which have a diameter of less than 0.5 cm and can, for example, still be curatively treated. This shows a further particular advantage of the method of the invention, since detection of such micrometastases is usually not possible with conventional diagnostic methods.

An analysis of the abovementioned types of cancer can also take place according to the invention by an in vitro diagnostic method. This can particularly preferably be used for establishing and/or optimizing the dose of the active compounds and/or substances to be administered, especially of the corresponding receptor ligands, for the subsequent therapy. The in vitro diagnostic method can include for example the following steps. The cells of a sample to be investigated, for example a sample resected surgically, are initially incubated with, for example, at least one ligand for the PPAR-y receptor, e.g. a thiazolidinedione, at least one ligand for the RAR and/or RXR receptor, e.g. trans-retinoic acid and at least one glucocorticoid, e.g. dexamethasone or dexamethasone acetate. Subsequently, cells thus prepared are treated with, for example, radioactive iodine and/or technetium. The conditions for this should permit uptake of the iodine or technetium by the cells and are known to the skilled worker. Finally, the content or the concentration of the transported iodine or technetium in the cells can be determined. Measurement of the radioactive iodine or technetium can be carried out by known methods, such as, for example, in a gamma counter. Alternatively, instead of incubation with a source of radioactive iodine or technetium and subsequent measurement of the radioactivity, it is also possible to measure expression of the NIS mRNA in the cells of the sample to be investigated after the treatment with the active compounds or ligands for the PPAR-y and RAR/RXR receptors in combination with glucocorticoid. In this case, the expression of NIS mRNA can be determined by means of a reverse transcriptase polymerase chain reaction (RT-PCR) or other methods known in the art.

A therapeutic method of the invention for treating the indicated types of cancer may include for example the following steps. Initially active compounds able to induce NIS gene expression in said types of tumors are administered to the patient to be treated. It is particularly preferred to administer, in particular orally and/or parenterally, for this purpose at least one ligand for the PPAR-y receptor, e.g. a thiazolidinedione, at least one or more ligands for the RAR and/or RXR receptor, e.g. trans-retinoic acid, in combination with at least one glucocorticoid, e.g. dexamethasone or dexamethasone acetate. Intravenous administration of the active compounds is likewise possible. The ligands and the glucocorticoid can be administered simultaneously or sequentially. After an appropriate time during which the NIS gene is induced, a suitable dose of a substance intended to bring about radiolysis of the tumor cells is administered. Radioactive iodide, e.g. ¹³¹iodide, is particularly preferred in this connection. Neither in the therapy nor in the described diagnostic methods is it absolutely necessary to wait an appropriate time before administering for example radioactive iodine. It is likewise possible for the administration of the ligands and glucocorticoid and the administration of iodine for example to take place simultaneously. The described steps are repeated if necessary at suitable time intervals, e.g. between 7 and 14 days. The radioiodide uptake takes place according to the invention organ-specifically, mainly in the cells of the indicated types of tumors, in which NIS expression is potentiated and iodide uptake is enhanced through the treatment. In contrast to a systemic radiotherapy known in the art, the tumor cells of the specified carcinomas and metastases are addressed specifically with the therapeutic method of the invention. The progress and success of treatment can be followed for example using the in vivo and/or in vitro diagnostic methods described above. The therapy of the invention can particularly advantageously take place after surgical removal of primary tumors, in order thus to be able to control metastases successfully. In some circumstances, it may also be preferred to employ the therapy of the invention instead of an operation or possibly before an operation. This is advantageous especially when an operation does not come under consideration for any reasons.

The present invention additionally relates to pharmacological compositions and products comprising active compounds able to induce, stimulate and/or enhance NIS gene expression in said types of tumors. Such compositions preferably comprise as active compounds ligands for the PPAR, especially PPAR-y, and RAR and/or RXR receptors. Compositions advantageous in this connection are those comprising at least one active compound from the class of thiazolidinediones and retinoic acid and/or its derivatives, especially trans-retinoic acid. It is additionally possible for such compositions according to the invention to include further substances, preferably activators, especially coactivators, of the NIS gene and/or histone deacetylase inhibitors, which likewise have a stimulating effect on NIS expression. In this regard, reference is made to the above description. The compositions or products may additionally include at least one pharmacologically acceptable carrier and/or excipient. Such compositions or products can be employed according to the invention as diagnostic aids and/or as medicaments for diagnosing and/or treating primary carcinomas and/or metastases, especially glandular carcinomas, preferably carcinomas of the thyroid, of the salivary gland, of the uterus and/or carcinomas of the breast, and the corresponding metastases.

The invention further includes a kit, in particular for the diagnosis and/or therapy of NIS gene-expressing carcinomas and/or metastases, which comprises active compounds able to induce, stimulate and/or enhance NIS gene expression in the tumors. This kit particularly preferably comprises at least one ligand for the PPAR-y receptor, in particular a thiazolidinedione such as, for example, ciglitazone, at least one ligand for the RAR and/or RXR receptor, in particular a trans-retinoic acid, and at least glucocorticoid, in particular dexamethasone or dexamethasone acetate. The kit may additionally comprise a source of radioactive iodine and/or technetium. The kit may additionally comprise substances having a modulating effect, preferably activators, in particular coactivators. In this regard, reference is made to the description above. Particularly preferred as activator in this connection is a histone deacetylase inhibitor, in particular trichostatin A or a butyrate. Concerning further features of the composition and products of the invention, reference is likewise expressly made to the description above.

Finally, the invention includes a use of at least one PPAR ligand, in particular a PPAR-y ligand, of at least one RAR and/or RXR ligand for producing a diagnostic composition and of at least glucocorticoid for the detection or for producing a medicament for the treatment of carcinomas and/or metastases which express an NIS gene. These carcinomas and/or metastases are in particular primary tumors and/or metastases of glandular carcinomas, in particular of salivary gland carcinomas, thyroid carcinomas, uterine carcinomas and/or carcinomas of the breast.

In a preferred embodiment of this aspect of the invention, the at least one PPAR-y ligand comprises one or more thiazolidinediones, in particular ciglitazone, pioglitazone and/or rosiglitazone and/or triglitazone and/or combinations of these as well as several synthetic ligands of PPAR-y. The at least one RAR and/or RXR ligand is advantageously a retinoic acid, in particular all-trans RA (atRA;Tretionin), 9-cis RA (Alitretinoin) and/or 13-cis RA (Isotretinoin), as well as suitable synthetic ligands of the retinoic acid receptor (RAR), or a pharmacologically/diagnostically acceptable derivatives thereof?. An appropriate pharmacologically acceptable derivative is advantageously a salt and/or an ester of retinoic acid, in particular an ester with an alkanoic acid, preferably having 1 to 4 C-atoms, or an ester with an inorganic acid. The glucocorticoid is selected from natural glucocorticoids such as cortison and cortisol, synthetic halogenated glucocorticoids such as amcinonid, alclometasone beclomethasone, betamethasone, clobetasone, clobetasole, cloprednole, clocortolone, dexamethasone, desoximetasone, diflucortolone, fluprednidene, fluocinolone, fluocortolone, flumethasone, halometasone, halcinonide, mometasone, triamcinolone, and synthetic non-halogenated glucocorticoids such as budesonide methylprednisolone, prednicarbate, prednisone, prednisolone, prednicarbate, prednisone, prednisolone, rimexolone, preferably the glucocorticoid is dexamethasone, dexamethasone acetate or disodium dexamethasone phosphate.

A particularly preferred embodiment of the use according to the invention provides for employing the diagnostic composition and/or the medicament for combination with a substance which is actively transported by the NIS symporter. Expression of the NIS gene is induced or stimulated and/or enhanced by the use of the described ligands, so that ultimately an increased activity of this symporter in the affected cells, and thus an enhanced uptake of iodine or of other substances for which the symporter has an affinity is to be observed. This enhanced activity of the symporter is utilized according to the invention for diagnosis or therapy by detecting the uptake of the affinity substance into the cell, or by bringing about through the uptake of, for example, radioactive affinity substances a radiolysis and thus a destruction of the affected cells. The affinity substances claimed are in particular halides, and in this connection particularly preferably iodine. A further preferred affinity substance is, for example, technetium.

In a particularly preferred embodiment of this aspect of the use according to the invention, the radioactive substance is radioactive iodine, preferably ¹²³I, ¹²⁵I and/or ¹³¹I. It is particularly preferred for the iodine, in particular the radioactive iodine, to be in the form of an iodide, preferably in the form of an alkali metal and/or alkaline earth metal iodide. Sodium iodide (NaI) is particularly advantageous in this connection.

In a further advantageous embodiment of the uses according to the invention, the diagnostic composition or the medicament is configured so that the at least one RAR/RXR ligand is administered first, the at least one PPAR ligand is administered second, and at least one glucocorticoid being administered after an appropriate time. Such a sequential administration has the advantage that the synergistic effect of the two ligands or of the two ligand groups in combination with glucocorticoid can be enhanced, which may derive in particular from initial suppression of the PPAR-y receptor in its activity for example through the presence of suppressors. The activation of RAR/RXR by the appropriate ligand(s) also converts PPAR-y into an active form, so that the full synergistic effect can be achieved through the subsequent administration of ligands for PPAR-y in combination with glucocorticoid. On the other hand, it may also be advantageous initially to administer at least one PPAR ligand, in particular at least one PPAR-y ligand, and later to administer at least one RAR/RXR ligand in combination with glucocorticoid. Administration of the ligand groups in combination with glucocorticoid at the same time may also be preferred. Concerning further features of these uses according to the invention, reference is expressly made to the above description.

Besides the described the use of said ligands for producing a diagnostic composition or for producing a medicament, the invention likewise includes the use of at least one PPAR ligand, in particular a PPAR-y ligand, at least one RAR and/or RXR ligand and at least one glucocorticoid for the diagnostic detection or for the treatment of carcinomas and/or metastases which express an NIS gene. Concerning further features of this aspect of the invention, reference is made to the above description.

The described method of the invention for the diagnosis and therapy of the types of tumors indicated above, and the relevant compositions, products and uses have the advantage compared with known methods and compositions inter alia that they make specific identification of primary tumors and/or their metastases, and an efficient and tumor-specific radioiodine therapy, possible.

Further advantages, features and possible uses of the invention are described below by means of the exemplary embodiments with reference to the drawings. In this connection, the various features may in each case be implemented on their own or in combination with one another.

The field of application of the underlying invention are glandular carcinomas, preferably carcinomas which will express NIS and the use of a radiopharmaceutical will be crowned with success, especially mamma carcinomas.

The invention will be further explained in the following Examples which are, however, not to be construed as limiting the invention.

### Examples

### Materials and Methods

All test substances used for experimental purposes (synthetic all-trans-retinoic acid (atRA, Retinol), Pioglitazone (belonging to the synthetic thiazolidindiones TZDs or Glitazone), dexamethasone acetate (hereinafter shortly referred to as "Dexa", belonging to the family of glucocorticoids) and docosahexaenoic acid (DHA) were each produced in the clean rooms of the Hubertus Labor in Freiburg, Germany using DMSO (Dimethylsulfoxid) as the solvent, and were used in the experiments without further purification.

The MCF-7 cell line, as well as the HELA cell line was purchased commercially from DSMZ (Braunschweig, Germany) with appropriate documentation.

Culture media (RPMI 1640 and Leibovitz's L15 medium), additives, and reagents for cell culture (glutamax, trypsin, PBS, EDTA, fetal bovine serum (FBS), 1% penicillin/streptomycin (P/S), L-glutamine) were purchased from Life Technologies (Darmstadt, Germany) or Invitrogen (Karlsruhe, Germany), respectively.

[¹³¹I]iodide (I-131) was purchased from Amersham Healthcare (Braunschweig, Germany), while [¹²⁴I]iodide (I-124) was produced at the cyclotron of the Interdisciplinary PET Center Würzburg, Germany, according to a standard procedure for preclinical and clinical applications described by Lamparter et al. (2014) and made available for use in cell experiments.

Other chemicals and solvents used (including acetone, ethanol, diemethyl sulfoxide (DSMO)) were purchased from Merck (Darmstadt, Germany) or Sigma-Aldrich (Deishofen, Germany), respectively.

Cell culture flasks 75 ml with filter were purchased from Greiner bio-one (Frickenhausen, Germany); sterile filter 0.22 µm pore size from Millipore (Carrigtwohill Cork, Ireland); and centrifuge tubes 15 ml and 50 ml from Sarstedt (Nürnbrecht, Germany).

The following equipments were used in cell culture and cell experiments:
An activimeter from Wellhofer Dosimetrie (Schwarzenbruck, Germany) for determining the radioactivity; Hamiliton Syringes from SGE Micro Volume Syringes (Darmstadt, Germany); the gamma-counter 1480 Wizard-Wallac from Perkin Elmer (Rodgau-Jügesheim, Germany) for determination of I-124/I-131 activity taken up by tumor cells after incubation; a hera cell incubator from Heraeus Instruments (Hanau, Germany) for cultivation and incubation of cells with I-124/I-131, with or without drug treatment; the sterile bench Hera Safe HS 12, Heraeus Instruments (Hanau, Germany) for all work with cells; a cryotank from Nunc (Roshilde, Denmark) for storage and preservation of cell lines; light microscopes Leica DMIL (Solms, Germany) with objectives 40 / Achrostigmat 40 × / 0.55 PH2, (Carl Zeiss, Oberkocher, Germany) and Leica C Plan 10 × / 0.22 PH1, with occulare Leica 10 × / 18 (Solms, Germany); sterile pipettes 5 ml, 10 ml, 25 ml from Eppendorf (Hamburg, Germany); reaction vessels (Epis) 1 ml and 1.5 ml from Sarstedt (Nürnbrecht, Germany); a sample shaker IKA MS1 MiNIShaker (Staufen, Germany); a water bath from Memmert GmbH (Schwabach, Germany); centrifuges: Universal 30F from Hettrich (Tuttlingen, Germany) and Megafuge 2.0R from Heraeus Instruments (Hanau, Germany); a Neubauer counting chamber; and a refrigerator-freezer from Liebherr (Ochsenhausen, Germany).

General cell culture preparation: All breast cancer cell lines used, notably MCF-7, are so-called primary human breast cancer cell lines (i.e., they originate from tumor tissues of patients with breast cancer or their metastases). Except for of the MCF-7 cell line, the utilized breast cancer cell lines possess only moderately weak or no NIS expression, such as the HeLa cell line, which is a non-NIS expressing Zervical cancer cell line that served as a negative control in the experiments underlying the invention.

The commercially purchased MCF-7, and HeLa cell lines were delivered in refrigerated cryotubes and stored in a cryotank with liquid nitrogen at -80 °C.

Prior to culture, the cells needed to be thawed. For this purpose, cryotubes were removed from the nitrogen tank, thawed in a 37 °C water bath, and the cell suspension was taken up briskly in 10 ml medium. The cell suspension was centrifuged at 1600 rpm for 5 min and the medium containing DSMO was discarded. The cell pellets were resuspended in fresh medium, transferred to a 75 ml cell culture flask and transferred to the incubator.

Cells were cultured at 37 °C under 5% CO₂ atmosphere in a steam humidified incubator. Every 2-3 days the cells were passaged, which included removal of the culture medium from the culture bottle, washing the cells once with PBS and then being overlaid with 1 ml of trypsin/EDTA. The culture bottle was gently swirled until the entire bottom of the bottle was covered. Excess trypsin/EDTA was removed, and the cells were placed in a blood cabinet at 37 °C for 5 min, depending on the cell line. Trypsin/EDTA was inactivated with 3 ml medium, and the cells were detached from the bottom of the bottle by pipetting up and down several times.

Depending on the cell line, cells were split 1:3 / 1:4 (MCF-7) or 1:5 / 1:6 (HeLa cell lines), seeded in new cell culture flasks and re-cultured or examined. All cell lines were mycoplasma-free and tested regularly in this regard. Cell counting was performed in a Neubauer counting chamber.

Cells were grown in Leibovitz's L15 medium to which 15% fetal bovine serum (FBS), 1% penicillin/streptomycin (P/S) were added per 500 ml each. The MCF-7 and HELA cells were cultured in RPMI 1640 medium with 10% FBS, 1% P/S, and 1% glutamax at 37 °C in an incubator (Hera cell from Heraeus Instruments; Hanau, Germany) under 5% CO₂ atmosphere.

### Pretreatment of the maliqen test cells:

Prior to the actual cell experimental applications, the test substances 1 µM atRA, and 0.1 µM Dexa each, as well as the combinations (1) 1 µM atRA, 0.1 µM Dexa (> 98 %) dissolved in DSMO (0.1 %), 10 µM Pioglitazone (Pio) and 10 µM DHA900EE; (2) 1 µM atRA and 10 µM Pioglitazone; 1 µM atRA + 10 µM DHA900EE in 1% DMSO; plus the combinations 1 µM atRA + 0,1 µM Dexa + 10 µM Pio, 1 µM atRA + 10 µM Pio + 10 µM DHA900EE, 1 µM atRA + 0,1 µM Dexa + 10 µM DHA900EE, 1 µM atRA + 0,1 µM Dexa + 10 µM Pio +10 µM DHA900EE in 2% DMSO dissolved. The corresponding stock or nutrient solutions were then prepared, aliquoted and stored at -20 °C. Confluent cell passages were washed with 2.7 ml PBS, detached with 1 ml trypsin-EDTA solution (0.05% in PBS) and resuspended in the cell culture medium. The cell suspension was seeded into 13 ml of drug-supplemented medium that contained differential combinations of the test compounds used, according to the protocol, to a total concentration of 10⁻⁵ M to 10⁻⁷ M of each drug component in a final volume of 14 ml of cell medium. The cells were then incubated in the respective with the mono-test substances as well as in their above-mentioned different combinations for 24 h, 48 h, or 72 h, respectively, with the medium being replaced every 24 h with fresh medium enriched with the test substances. It turned out that a 48-h incubation period was completely sufficient.

Statistical analysis: Statistical comparisons were performed using GraphPad Prism version 8.1 for Windows (GraphPad Software, La Jolla, CA, USA), with a significance level of P value <0.05. Comparison between groups was determined by performing a one- or two-factor analysis of variance (ANOVA) including post hoc multiple comparison analysis (Tukey's honestly significant difference test, Dunnett's method, or Bonferroni multiple comparison test, as appropriate). The curves shown in the graphs were polynomic interpolated by nonlinear regression based on an allosteric sigmoidal equation fitting model.

### Example 1: Cell Uptake Studies

The optimum number of cells was determined for each cell line in preliminary tests. All incubations took place in the blood cabinet at 37 °C and 5% CO₂ atmosphere. Before the examination, the culture medium was aspirated from the culture bottle with the tumor cells, which had completely overgrown according to visual inspection, under a sterile bench, the cells were trypsinized with trypsin/EDTA and briefly centrifuged for 5 min at 1600 rpm in a 50 ml Falcon tube. The trypsin-EDTA supernatant was pipetted off, the cells were resuspended in protein-free medium, and the exact number of cells was determined using a Neubauer counting chamber. Cell suspensions consisting of 4-4.5×10⁵ tumor cells in 500 µl PBS (5% BSA) were prepared for each individual experiment. 30 mg of sodium or also [TZYQ)] vinegar dissolved in PBS (5% BSA) with a preset activity concentration, generally with 100000-250000 cpm were added to the cell suspensions. The samples were incubated for 5, 15, 30, 60 and 90 min at 37 °C and 5% CO₂ atmosphere in the incubator.

The recordings from the [¹²⁴I]iodide and [¹⁸F] tetrafluoroborate ([¹⁸F]TFB; not shown) were examined in dependence of the incubation time (5-90 min), respectively in MCF-7 -cellsand HELA-cells, which were stimulated with different combinations consisting of the used, synthetic mono-test substance classes (Retinol (here: atRA), Thiazolidindione/Glitazone (here: Pioglitazone), Glucocorticoid (here: dexamethasone), Omega-3 fatty acids ethyl ester (here: Docosahexaenoic acid 900EE/DHA900EE ) (24-48 h) were pre-stimulated at 37 °C for expression of hNIS protein on the cell surface, i.e., in all cell lines and were compared with results from non-stimulated cells.

After the specified incubation time, samples were immediately quenched on ice and centrifuged. The liquid supernatant was pipetted off, and each sample was washed twice with 500 µl ice-cold PBS (5% BSA) and centrifuged again. Radioactivity in the cell pellets was determined in a well-calibrated gamma counter (Wizard 2480, Perkin-Elmer, Rodgau, Germany). Cell-associated fractions were calculated as a percentage of total activity added per number of cells.

All experiments were performed in at least triplicate and repeated independently on at least three different days.

### Example 2: Inhibition Experiments

The inhibitory effect of the non-radioactive NIS substrates NaBF₄, potassium fluorosulfate (KSO₃F), potassium iodide (KI) iodide (KI) and potassium iodate (KIO₃) on the uptake of [¹²⁴I]iodide in MCF-7 cells was investigated and compared with the control experiment performed in parallel and confirmed in several experiments. [¹²⁴I]iodide from the manual synthesis procedure and later also from the module-based synthesis (see above) was used. Cells were separated, counted, and prepared as described above. PBS-buffered stock solutions of NaBF₄, KSO₃F, KI, and KIO₃, each at a concentration of 10⁻³ M, were added separately to the cell suspension samples (samples in triplicate) at a final concentration of either 1 µM, 10 µM, or 100 µM for each blocking reagent. All samples were carefully shaken and incubated at 37 °C for 30 min. After adding 50 µl of the freshly formulated [¹²⁴I]iodide tracer (100-120 kBq/1 ml) to each sample, the tubes were shaken well and incubated for an additional 60 min. All samples were processed in parallel and gamma counted as described above.

### Example 3: Reduction of Iodide Efflux.

However, in order to achieve a therapeutic effect of the radiopharmaceutical and the desired DNA damage to the tumor cells, it must be possible to prolong the retention time in a stable and predictable manner. In order to additionally delay the iodide efflux and to enhance the associated effect of the radiopharmaceutical on the cells, the anion channel blocker DIDS (4.4'-diisathiocyano-2.2'-stilbene disulfonic acid) should preferably be applied to the patient beforehand.

Results of the cell experiments: The focus of the underlying idea was to find or evaluate the most effective stimulatory test substance of Retionid X Receptors (RXR) as well as that of PPAPγ or the most effective stimulatory test substance combination(s) of the above mentioned used test substances, which in the used malignant cell lines can guarantee in vitro and/or in vivo diagnostics (using [¹²⁴I]iodide (I-124)]iodide (I-124)]iodide/[¹⁸F] tetrafluoroborate, [¹⁸F]TFB) and/or therapy ([¹³¹I]iodide [I-131]iodide) can be guaranteed. As a result, it can be stated that it is exclusively the malignant breast cancer cell line MCF-7, which by nature has a very high stimulatory capacity, especially amplifying triggered by the used, specifically acting test substances (ligand), and thus induces a rapid NIS expression, which fulfills the cardinal objective - an in vivo and/or in vitro diagnostic and/or therapeutic success guaranteed.

The HeLa cell line were stimulated with the same agents as the malignant breast cancer cell line (MCF-7) and the result clearly shows that the desired ligand-stimulating effect on NIS expression with its corresponding radioiodide uptake ([¹²⁴I]iodide) was not successful. Furthermore, HELA cell lines, which is a non-NIS MYBQ cell line, were used as a negative control for the Specificity verification of the test substances (agents) used in connection with NIS expression.

With the help of the malignant breast-cancer cell lines investigated in addition to the MCF-7 cell line in focus, the specificity of the stimulating test substance (synthetic ligands) or test substance combinations used was also impressively demonstrated in the underlying idea.

In the individual cell line experiments, the same test substance concentrations were always dissolved in the different solvent concentrations DSMO; specifically: 1 µM atRA, 0.1 µM Dexamethasone (Dexa) as well as the combination 1 µM atRA + 0.1 µM Dexa in 0.1 % DSMO plus 10 µM Pioglitazone (PIO), 10 µM DHA900EE and the combinations 1 µM atRA + 10 µM PIO, 1 µM atRA + 10 µM DHA900EE in 1% DSMO plus the combinations 1 µM atRA + 0.1 µM Dexa + 10 µM PIO, 1 µM atRA + 10 µM PIO + 10 µM DHA900EE, 1 µM atRA + 0.1 µM Dexa + 10 µM DHA900EE, 1 µM atRA + 0.1 µM Dexa + 10 µM PIO +10 µM DHA900EE in 2% DSMO and injected into the described nutrient liquid.

Further advantages, features of the underlying invention are described below on the basis of various embodiment examples with reference to the drawings. Here, the different features may be realized individually or in combination with each other. The following experimental results are shown in the following figures:

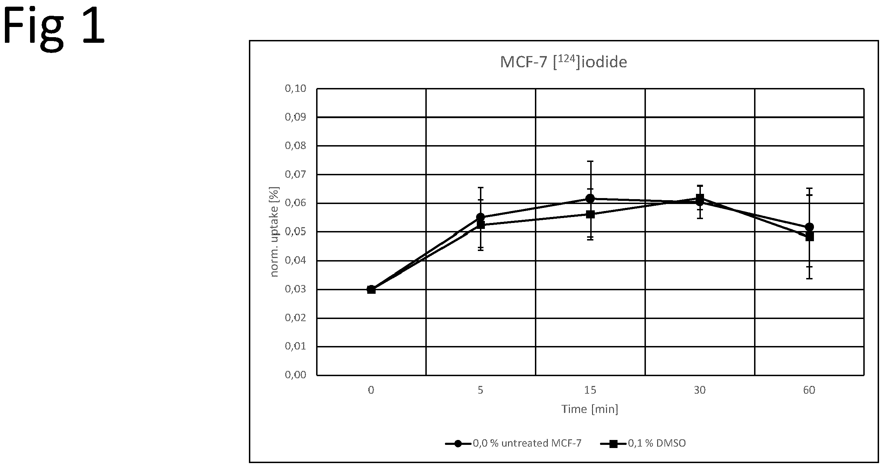

Fig. 1 shows untreated MCF-7 cells, as well as MCF-7 cells pretreated with DSMO acting as a solvent at a concentration of 0.1% DSMO (solvent), and their iodide uptake assay after an incubation time of max 90 min. Values were expressed as mean +/- SD (n ≥ 3).

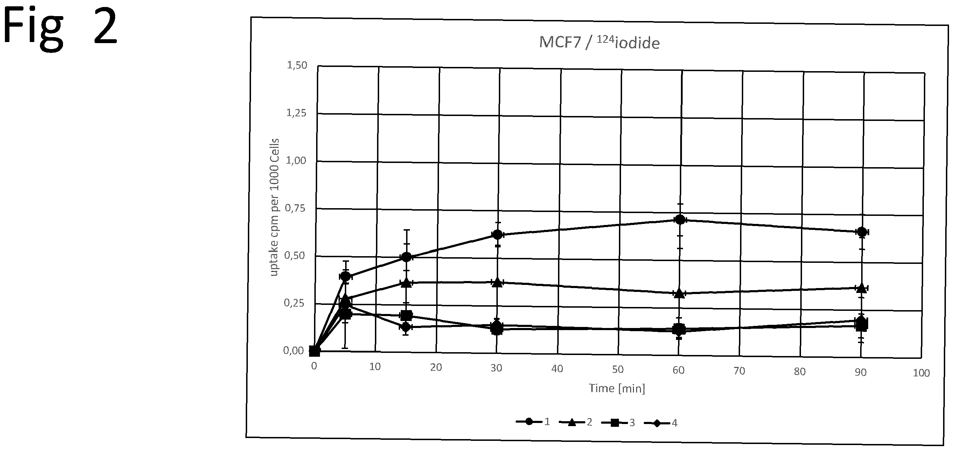

Fig. 2 shows the cell-specific effect of ATRA or Dexa, Pioglitazone (PIO) or DHA900EE, and the solvent DSMO on iodide uptake in MCF-7 cells. MCF-7 cells were pretreated with 1 µM atRA or with 0.1 µM Dexa 10 µM Pioglitazone (PIO), or with 10 µM DHA900EE for 48 h in culture medium. The iodide uptake assay was performed for a maximum of 90 min, values are expressed as mean +/- SD (n ≥ 4).

Fig. 3 shows the cell-specific effect of various combinations of the mono-test substances atRA or Dexamethasone (Dexa), Pioglitazone (Pio) or DHA900EE on iodid uptake in MCF-7 cells. The MCF-7 cells were treated with 1 µM atRA + 0.1 µM Dexa, 1 µM atRA + 10 µM Pio, 1 µM atRA + 10 µM DHA900EE, 1 µM atRA + 0.1 µM Dexa + 10 µM PIO, 1 µM atRA + 10 µM PIO + 10 µM DHA900EE, 1 µM atRA + 0.1 µM Dexa + 10 µM DHA900EE, 1 µM atRA + 0.1 µM Dexa + 10 µM Pio +10 µM DHA900EE pre-treated in nutrient liquid for 48 h. The iodide uptake assay was performed for a maximum of 90 min, values are expressed as mean +/- SD (n ≥ 4).

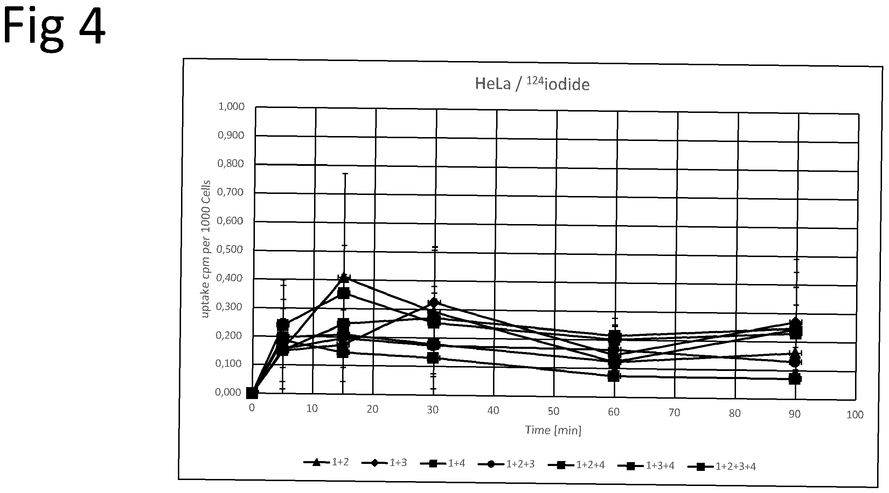

Fig. 4 shows the cell-specific effect of various combinations of the mono-test substances atRA or dexamethasone, pioglitazone (Pio) or DHA900EE on iodide uptake in Hela cells. Hela cells were treated with 1 µM atRA + 0.1 µM Dexa, 1 µM atRA + 10 µM Pio, 1 µM atRA + 10 µM DHA900EE, 1 µM atRA + 0.1 µM Dexa + 10 µM Pio, 1 µM atRA + 10 µM Pio + 10 µM DHA900EE, 1 µM atRA + 0.1 µM Dexa + 10 µM DHA900EE, 1 µM atRA + 0.1 µM Dexa + 10 µM Pio +10 µM DHA900EE pre-treated for 48 h in a nutrient liquid. The iodide uptake test was performed for max. 90 min. Values were expressed as mean +/- SD (n ≥ 4).

Conclusion: We assume that the synergistic effect of NIS expression is due to the additional application of dexamethasone acetate, and the associated uptake of the radioiodide (here the radioiodide [¹²⁴I]iodide(I-124), where the NIS "absorbs" all radiopharmaceuticals) did not result from their direct stimulation of the NIS gene, which occurred through the specific ligands of the PPAR-γ and the RX-receptors, but rather through the translocation of the formed in the cytoplasm to the basal/cell surface membrane NIS in the MCF-7 cell, because only a quantitative increase of NIS in the basal membrane preprogrammed a significantly increased uptake of radioiodide. With the underlying results and the surprisingly high iodide uptake documented *in vitro,* it is expected that a high radiation dose greater than 90 mCi ¹³¹Iodide, which is currently for the effective treatment of target tumors *in vivo,* can be significantly reduced so to therewith significantly reduce the side effect profile of a Gamma radiation treatment.

In our cell line experiments the radiopharmaceutical [¹²⁴I]iodide (I-124) was utilized, since it is suitable for the diagnostic imaging (scintigraphy) through its delta radiation, while the [¹³¹I]iodine (I-131) is used only for therapeutic purposes through its gamma radiation, so that the DNA damage can take place in the malignant cells and the cell death directly connected to them. Likewise, one must know that [¹²⁴I]iodide (I-124) and [¹⁸F]tetrafluoroborate are comparable radiopharmaceuticals; concerning the latter it is referred to the comparative study in S. Samnick et al., Clin Nucl Med 43: 162-167 (2018), entitled: Initial Clinical Investigation of [¹⁸F]tetrafluoroborate PET/CT in Comparison to [¹²⁴I]iodine PET/CT for Imaging Thyroid Cancer.

## Claims

**1.** A diagnostic composition, therapeutic composition or medicament for use in the *in vivo* diagnosis or treatment of NIS gene-expressing carcinomas and/or metastases of carcinomas by stimulating or enhancing NIS gene expression in cells of the carcinomas or the metastases thereby increasing NIS-symporter function, said composition comprising the components:
(a) at least one Peroxisome Proliferator-Activated Receptor-y (PPAR-y) ligand,
(b) at least one Retinoic Acid Receptor (RAR) and/or Retinoid X Receptor (RXR) ligand,
(c) at least one glucocorticoid, or a salt and/or ester thereof, and
(d) a radioactive entity actively transported by the NIS symporter into cells of the carcinomas or the metastases.

**2.** The diagnostic composition, therapeutic composition or medicament for use in the *in vivo* diagnosis or treatment of NIS gene-expressing carcinomas and/or metastases of carcinomas of claim 1, wherein the NIS gene-expressing carcinomas and/or metastases of carcinomas are primary tumors of glandular carcinomas, preferably salivary gland carcinomas, thyroid carcinomas, uterine carcinomas or carcinomas of the breast, most preferably are carcinomas of the breast.

**3.** The diagnostic composition, therapeutic composition or medicament for use in the *in vivo* diagnosis or treatment of NIS gene-expressing carcinomas and/or metastases of carcinomas of claim 1 or 2, wherein the component (a) is a thiazolidinedione, preferably is selected from ciglitazone, pioglitazone, rosiglitazone, troglitazone and mixtures thereof, most preferably is pioglitazone.

**4.** The diagnostic composition, therapeutic composition or medicament for use in the *in vivo* diagnosis or treatment of NIS gene-expressing carcinomas and/or metastases of carcinomas in any one of claims 1 to 3, wherein the component (b) all-trans RA (=tRA;Tretionin) and/or and/or 9-cis RA (Alitretinoin) and/or 13-cis RA (Isotretinoin) as well as several synthetic ligands of retinoic acid receptor (RAR)is retinoic acid or a salt or an ester thereof, preferably the retinoic acid is trans-retinoic acid or a C₁₋₄ alkanoic acid ester thereof.

**5.** The diagnostic composition, therapeutic composition or medicament for use in the *in vivo* diagnosis or treatment of NIS gene-expressing carcinomas and/or metastases of carcinomas in any one of claims 1 to 4, wherein the component (c) is selected from natural glucocorticoids such as cortison and cortisol, synthetic halogenated glucocorticoids such as amcinonide, alclometasone beclomethasone, betamethasone, clobetasone, clobetasole, cloprednole, clocortolone, dexamethasone, desoximetasone, diflucortolone, fluprednidene, fluocinolone, fluocortolone, flumethasone, halometasone, halcinonide, mometasone and triamcinolone, and synthetic non-halogenated glucocorticoids such as budesonide methylprednisolone, prednicarbate, prednisone, prednisolone, prednicarbate, prednisone, prednisolone and rimexolone, or a salt and/or ester thereof, preferably the glucocorticoid is dexamethasone, dexamethasone acetate or disodium dexamethasone phosphate.

**6.** The diagnostic composition, therapeutic composition or medicament for use in the *in vivo* diagnosis or treatment of NIS gene-expressing carcinomas and/or metastases of carcinomas in any one of claims 1 to 5, wherein the radioactive entity (d) is a radioactive substance, preferably contains a radioactive halogen or radioactive halogenide, most preferably the radioactive substance contains ¹⁸F, ¹²³I, ¹²⁴I, ¹²⁵I or ¹³¹I, or [¹⁸F] tetrafluoroborate.I

**7.** The diagnostic composition, therapeutic composition or medicament for use in the *in vivo* diagnosis or treatment of NIS gene-expressing carcinomas and/or metastases of carcinomas in any one of claims 1 to 6, wherein the diagnostic composition, therapeutic composition or medicament further comprises
(i) saturated or unsaturated fatty acids or pharmaceutically acceptable salts or esters thereof, preferably docosahexaenoic acid or docosahexaenoic acid ethylester;
(ii) pharmaceutically or diagnostically acceptable carriers, solvents, stabilisators and masking agents.

**8.** The diagnostic composition, therapeutic composition or medicament for use in the *in vivo* diagnosis or treatment of NIS gene-expressing carcinomas and/or metastases of carcinomas in any one of claims 1 to 7, wherein the diagnostic composition, therapeutic composition or medicament
(i) is in the form of a single dose application and contains at least all components (a) to (c), while the radioactive entity being contained in same or different dosage form; or
(ii) is in the form of a multiple dose application, wherein the at least one RAR ligand and/or RXR ligand is contained in a first dosage form for being administered first, and the at least one PPAR-y ligand and the at least one glucocorticoid is/are present in separate second, and optionally third, dosage form(s) for being administered after an appropriate time ranging from about some hours to some days after the administration of the first dosage form, preferably the appropriate time is about 1 to about 3 days.

**9.** The diagnostic composition, therapeutic composition or medicament for use in the *in vivo* diagnosis or treatment of NIS gene-expressing carcinomas and/or metastases of carcinomas in any one of claims 1 to 8, which is a diagnostic composition and is used for diagnosis of carcinomas and/or metastases of carcinomas, wherein the diagnosis comprises
(a) application of the diagnostic composition as defined in claims 1-8 to a patient in need of such diagnosis;
(b) detecting the accumulation of the radioactive entity (d) in the body region of the patient suspected to contain the carcinomas or metastases by radio-detection;
(c) evaluating and comparing the radioactive signals with the radioactive signal of a healthy patient or an earlier signal of the same patient; and
(d) determining the severity or change of carcinomas or presence of metastases in the patient.
9. The diagnostic composition, therapeutic composition or medicament for use in the *in vivo* diagnosis or treatment of NIS gene-expressing carcinomas and/or metastases of carcinomas in any one of claims 1 to 8, which is a diagnostic composition for detecting/diagnosing metastases with a diameter of less than about 0.5 cm.

**10.** A method for *in vivo* and *ex vivo* diagnosis of NIS gene-expressing carcinomas and/or metastases of carcinomas by stimulating or enhancing NIS gene expression in cells of the carcinomas or the metastases thereby increasing NIS-symporter function, said method comprises administering a patient or subjecting a tissue sample in need of such diagnosing a composition comprising the components:
(a) at least one Peroxisome Proliferator-Activated Receptor-y (PPAR-y) ligand,
(b) at least one Retinoic Acid Receptor (RAR) and/or Retinoid X Receptor (RXR) ligand,
(c) at least one glucocorticoid, , or a salt and/or ester thereof, and
(d) a radioactive entity actively transported by the NIS symporter into cells of the carcinomas or the metastases.

**11.** A method for treating NIS gene-expressing carcinomas and/or metastases of carcinomas by stimulating or enhancing NIS gene expression in cells of the carcinomas or the metastases thereby increasing NIS-symporter function, comprising: administering to a patient in need thereof a composition comprising the components:
(a) at least one Peroxisome Proliferator-Activated Receptor-y (PPAR-y) ligand,
(b) at least one Retinoic Acid Receptor (RAR) and/or Retinoid X Receptor (RXR) ligand,
(c) at least one glucocorticoid, , or a salt and/or ester thereof, and
(d) a radioactive entity actively transported by the NIS symporter into cells of the carcinomas or the metastases,
to stimulate or enhance uptake of the radioactive affinity containing substance actively transported by the NIS symporter by induction of NIS gene expression in the cells of the patient.

**12.** The method of claim 10 or 11, wherein the NIS gene-expressing carcinomas, metastases of carcinomas or both thereof are selected from the group consisting of primary tumors, metastases of glandular carcinomas, salivary gland carcinomas, thyroid carcinomas, uterine carcinomas, and carcinomas of the breast.

**13.** The method of any one of claims 10 to 12, wherein the compositions and the compounds (a) to (d) are as defined in claims 3 to 8.
